# EUROPÄISCHE PATENTSCHRIFT

(11) **EP 0 444 280 B1**
(45) Veröffentlichungstag und Bekanntmachung des Hinweises auf die Patenterteilung: **27.07.1994**
(21) Anmeldenummer: 90124395.6
(22) Anmeldetag: 17.12.1990
(51) Int. Cl.: C23C 14/08, C23C 14/32, A61L 27/00, A61F 2/28

(54) **Verfahren zur Herstellung von physiologisch verträglichen Oxidschichten auf Skelettimplantaten**
Process for the production of physiologically compatible oxide layers on bone implants
Procédé de production de couches oxydiques physiologiquement compatibles sur les implants d'os

(30) Priorität: 23.02.1990 DE 4005692
(43) Veröffentlichungstag der Anmeldung: 04.09.1991
(73) Patentinhaber: O.M.T. OBERFLÄCHEN UND MATERIALTECHNOLOGIE GMBH, 23569 Lübeck (DE)
(72) Erfinder: Repenning, Detlev, Dr., W-2057 Reinbek (DE); Thull, Roger, Prof. Dr. Ing., W-8700 Würzburg (DE)
(74) Vertreter: Niedmers, Ole, Dipl.-Phys.

(56) Entgegenhaltungen:
- DE-A- 1 943 118
- JAPANESE PATENTS GAZETTE, 14. August 1984, Kapitel: "Metallurgy", Seite 24; & JP-A-60 092 761
- JAPANESE JOURNAL OF APPLIED PHYSICS, Band 28, Nr. 5.II, Mai 1989, Seiten L816-L818, Tokyo, JP; H. ISHIWARA et al.: "Preparation of Yb-Ba-Cu-O superconducting films using an arc discharge evaporation method"
- APPLIED OPTICS, Band 28, Nr. 14, 15. Juli 1989, Seiten 2806-2808, Optical Society of America, New York, US; H.O. SANKUR et al.: "Deposition op optical thin films by pulsed laser assisted evaporation"

## Beschreibung

Die Erfindung betrifft ein Verfahren zur Herstellung von physiologisch verträglichen Oxidschichten auf Skelettimplantaten (Substraten) aus Metall oder Metallegierungen, wobei die Oxidschichten mittels eines Hochvakuum-Plasma-Beschichtungsverfahren auf dem Substrat aufgebracht werden.

Ein Verfahren dieser Art ist bekannt (Japanese Patent Gazette, 14.08.1984, Kapitel "Metallurgy", Seite 24; & JP-A-60-092761).

Grundsätzlich kommt es neben einer hohen Festigkeit bei Skelettimplantaten wesentlich darauf an, daß sie mit dem sie umgebenden biologischen Milieu verträglich sind. Es sind grundsätzlich seit langem Implantatwerkstoffe aus Metall oder Metallegierungen bekannt, die eine Oxidschicht aufweisen, die sich beispielsweise beim Einsatz des Implantats in das biologische Milieu in diesem mehr oder weniger zufällig ausbildet.

So sind beispielsweise bisher Skelettimplantate aus Titan unbeschichtet implantiert worden, wobei durch Reaktion im biologischen Milieu sich auf dem Titanwerkstoff eine Titandioxidschicht mehr oder weniger zufällig ausgebildet hat. Man hat aber auch versucht, durch anodischen Auftrag Titandioxidschichten auf Titanimplantaten vor der Implantierung auszubilden, wobei Titandioxidschichten mit einer Schichtdicke von ca. 4 x 10⁻⁷ bis 7 x 10⁻⁷ m aufgebaut wurden.

Es hat sich jedoch gezeigt, daß diese sich zufällig aufbauenden Schichten nur unzureichend dünn, porenreich und beschädigungsanfällig sind. Dieses gilt gleichermaßen für die anodisierten Titanoxidschichten. Ist eine Oxidschicht erst einmal verletzt, führt dieses zu Reibkorrosion und Bildung von Titanverbindungen mit niedriger Oxidationsstufe im Körper, die in der Regel nekrotisch wirken.

Es sind auch andere Verfahren bekannt, beispielsweise das sogenannte Plasmaspritzen, bei dem Oxide aufgespritzt werden. Diese haben im Einsatz ebenfalls den Nachteil der mangelnden Haftung und der Porosität gezeigt, die ebenfalls zur Korrosion des das Skelettimplantat bildenden Grundwerkstoffs mit allen seinen Problemen führen können.

Es ist Aufgabe der vorliegenden Erfindung, ein Verfahren zu schaffen, mit dem Oxidschichten auf Skelettimplantaten aufgebracht werden können, die besonders gute Integrationseigenschaften im biologischen Milieu aufweisen, wobei die Schicht porenfrei und gleichmäßig aufbaubar sein soll und mechanisch widerstandsfähig ist, und das verhältnismäßig leicht ausgeführt werden kann.

Gelöst wird die Aufgabe gemäß der Erfindung dadurch, daß die Oxidschichten aus multinären Refraktärmetallsystemen der Refraktärmetalle Titan, Zirkonium, Hafnium, Niob, Tantal, Wolfram oder Molybdän aufgebaut sind und mittels Hochvakuum-Plasma-Beschichtungsverfahren auf dem Substrat aufgebracht werden. Der Vorteil des erfindungsgemäßen Verfahrens liegt im wesentlichen darin, daß Oberflächen geschaffen werden, die wirklich porenfrei sind, so daß es nicht zu den nachteiligen Korrosionswirkungen wie bei den Skelettimplantaten bekannter Art kommt. So können beispielsweise multinäre Systeme wie Titan-Zirkoniumoxid die Oxidschicht bilden. Vorteilhafterweise kann aber auch für das multinäre System Titan-Nioboxid benutzt werden.

Gemäß einer vorteilhaften Ausgestaltung des Verfahrens wird als Beschichtungsverfahren das Lichtbogenverdampfungsverfahren verwendet. Bei diesem Verfahren handelt es sich um ein solches mit einem hohen Plasma-Metallanteil . Gleichermaßen vorteilhaft sind als Beschichtungsverfahren das Laserverdampfungsverfahren oder das sogenannte Wire-Explosion-Verfahren, die sich ebenfalls durch einen hohen Plasma-Metallanteil auszeichnen.

Die erfindungsgemäß ausgebildeten Oxidschichten weisen vorteilhafterweise eine Dicke zwischen 10⁻⁶ bis 2 x 10⁻⁵ m auf, die porenfrei ausgebildet werden kann.

Zum besseren Anwachsen im Knochen kann verfahrensgemäß auch eine definierte Mikrorauheit eingestellt werden, so daß dieses Verfahren zusätzlich noch eine besonders gute Knochenintegration neben der sehr guten biologischen Verträglichkeit der Oxidschicht liefert.

Es hat sich gezeigt, daß in vielen Fällen schon mit Schichtdicken zwischen 8 x 10⁻⁶ bis 10⁻⁵ m Dicke sehr gute Schichteigenschaften erreicht werden konnten.

Mit Schichten aus Titan-Zirkoniumoxid (Ti, Zr [O₂]) oder Titan-Nioboxid (Ti, Nb [O₂]) auf metallischen Skeletttimplantaten können hervorragende Eigenschaften aufweisende Implantate für den Hüft-, den Zahn- oder Schulterbereich erreicht werden. Die mit dem erfindungsgemäßen Verfahren aufgebrachte Schicht eignet sich auch besonders gut für die sogenannten kapselnden, körpernicht-verträglichen Legierungen aus CoCrNo oder ähnlichen Legierungen. Grundsätzlich kann die Oxidschicht aber auch auf ein Substrat aus Titan, Edelstahl oder beliebigen anderen Metallen, Metallverbindungen oder Legierungen aufgebracht werden.

## Patentansprüche

1. Verfahren zur Herstellung von physiologisch verträglichen Oxidschichten auf Skelettimplantaten (Substraten) aus Metall oder Metallegierungen, wobei die Oxidschichten mittels eines Hochvakuum-Plasma-Beschichtungsverfahren auf dem Substrat aufgebracht werden, dadurch gekennzeichnet, daß die Oxidschichten aus multinären Refraktärmetallsystemen der Refraktärmetalle Titan, Zirkonium, Hafnium, Niob, Tantal, Wolfram oder Molybdän aufgebaut sind.

2. Verfahren nach Anspruch 1, dadurch gekennzeichnet, daß das Beschichtungsverfahren das Lichtbogenverdampfungsverfahren ist.

3. Verfahren nach Anspruch 1, dadurch gekennzeichnet, daß das Beschichtungsverfahren das Laserverdampfungsverfahren ist.

4. Verfahren nach Anspruch 1, dadurch gekennzeichnet, daß das Beschichtungsverfahren das Wire-Explosion-Verfahren ist.

5. Verfahren nach einem oder mehreren der Ansprüche 1 bis 4, dadurch gekennzeichnet, daß das multinäre System aus Titan-Zirkoniumoxid gebildet wird.

6. Verfahren nach einem oder mehreren der Ansprüche 1 bis 4, dadurch gekennzeichnet, daß das multinäre System aus Titan-Nioboxid gebildet ist.

7. Verfahren nach einem oder mehreren der Ansprüche 1 bis 6, dadurch gekennzeichnet, daß die Oxidschicht zwischen 10⁻⁶ - 2 x 10⁻⁵ m dick ist.

8. Verfahren nach Anspruch 7, dadurch gekennzeichnet, daß die Oxidschicht zwischen 8 x 10⁻⁶ - 10⁻⁵ m dick ist.

## Claims

1. A process for the preparation of physiologically compatible oxide layers on skeletal implants (substrates) of metal or metal alloys, wherein the oxide layers are applied to the substrate by means of a high vacuum plasma coating process, characterized in that the oxide layers are built up from multinary refractory metal systems of the refractory metals titanium, zirconium, hafnium, niobium, tantalum, tungsten or molybdenum.

2. A process according to claim 1, characterized in that the coating process is the arc evaporation process.

3. A process according to claim 1, characterized in that the coating process is the laser evaporation process.

4. A process according to claim 1, characterized in that the coating process is the wire explosion process.

5. A process according to one or more of claims 1 to 4, characterized in that the multinary system is formed by titaniumzirconium oxide.

6. A process according to one or more of claims 1 to 4, characterized in that the multinary system is formed by titaniumniobium oxide.

7. A process according to one or more of claims 1 to 6, characterized in that the oxide layer is between 10⁻⁶ - 2 x 10⁻⁵m in thickness.

8. A process according to claim 7, characterized in that the oxide layer is between 8 x 10⁻⁶ - 10⁻⁵m in thickness.

## Revendications

1. Procédé de formation de couches d'oxydes physiologiquement compatibles et tolérées sur des implants pour os ou substrats en métaux ou alliages de métaux, couches qui sont déposées sur le substrat suivant un procédé de revêtement par plasma sous un vide élevé, procédé caractérisé en ce que les couches d'oxydes sont formées de systèmes mixtes de métaux réfractaires pris parmi le titane, le zirconium, l'hafnium, le niobium, le tantale, le tungstène et le molybdène.

2. Procédé selon la revendication 1, caractérisé en ce que le procédé de revêtement est le procédé par évaporation à l'arc électrique.

3. Procédé selon la revendication 1, caractérisé en ce que le procédé de revêtement est le procédé par évaporation au laser.

4. Procédé selon la revendication 1, caractérisé en ce que le procédé de revêtement est le procédé d'explosion par fil métallique.

5. Procédé selon une ou plusieurs des revendications 1 à 4, caractérisé en ce que le système mixte est formé d'oxyde de titane et zirconium.

6. Procédé selon une ou plusieurs des revendications 1 à 4, caractérisé en ce que le système mixte est formé d'oxyde de titane et niobium.

7. Procédé selon une ou plusieurs des revendications 1 à 6, caractérisé en ce que la couche d'oxyde a une épaisseur de 10⁻⁶ à 2 x 10⁻⁵m.

8. Procédé selon la revendication 7, caractérisé en ce que la couche d'oxyde a une épaisseur de 8 x 10⁻⁶ à 10⁻⁵ m.
